(19) **Europäisches Patentamt / European Patent Office / Office européen des brevets**

(11) **EP 4 715 384 A1**

(12) **EUROPEAN PATENT APPLICATION**
published in accordance with Art. 153(4) EPC

(43) Date of publication:
**25.03.2026 Bulletin 2026/13**

(21) Application number: **24807210.0**

(22) Date of filing: **14.05.2024**

(51) International Patent Classification (IPC):
***G01N 33/68*** (2006.01)

(52) Cooperative Patent Classification (CPC):
**G01N 33/68**

(86) International application number:
**PCT/JP2024/017844**

(87) International publication number:
**WO 2024/237258 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.05.2023 JP 2023081624**

(71) Applicants:
• **SHIMADZU CORPORATION**
 **Kyoto-shi, Kyoto 604-8511 (JP)**
• **ST. LUKE'S INTERNATIONAL UNIVERSITY**
 **Tokyo 104-0044 (JP)**

(72) Inventors:
• **AOKI, Yutaka**
 **Kyoto-shi, Kyoto 604-8511 (JP)**

• **WATANABE, Makoto**
 **Kyoto-shi, Kyoto 604-8511 (JP)**
• **SATO, Taka-Aki**
 **Kyoto-shi, Kyoto 604-8511 (JP)**
• **KIMURA, Takeshi**
 **Tokyo 104-0044 (JP)**
• **KOSHIZAKA, Takuya**
 **Tokyo 104-0044 (JP)**
• **OHTAKE, Jyunya**
 **Tokyo 104-0044 (JP)**
• **KUMAKURA, Yasuhisa**
 **Tokyo 104-0044 (JP)**

(74) Representative: **Müller-Boré & Partner**
 **Patentanwälte PartG mbB**
 **Friedenheimer Brücke 21**
 **80639 München (DE)**

(54) **METHOD FOR IDENTIFYING NON-ALCOHOLIC FATTY LIVER DISEASE, AND BIOMARKER**

(57) To provide a method for identifying NAFLD with excellent identification performance and a biomarker used in the method. An identification method for nonalcoholic fatty liver disease includes: an acquisition step of acquiring a sample derived from a body fluid of a subject; a detection step of detecting eight kinds of compounds consisting of alanine, isoleucine, glycine, serine, glutamic acid, asparagine, tyrosine, and uric acid in the sample; and a use step of using all detection amounts of the eight kinds of compounds obtained in the detection step.

FIG. 1

TRAINING SET (AREA UNDER CURVE = 0.86)

TEST SET (AREA UNDER CURVE = 0.86)

EP 4 715 384 A1

**Description**

TECHNICAL FIELD

The present invention relates to an identification method for nonalcoholic fatty liver disease (NAFLD) and a biomarker for use in the identification method.

BACKGROUND ART

NAFLD refers to fatty liver conditions not caused by alcohol, viral infection, or similar factors. NAFLD encompasses simple steatosis, in which fat just accumulates in hepatocytes, and nonalcoholic steatohepatitis (NASH), in which hepatocytes become damaged and lose function, leading to inflammation and fibrosis from fatty liver with a risk of progressing to cirrhosis or liver cancer. Since simple steatosis can progress to nonalcoholic steatohepatitis, the diagnosis of NAFLD, including NASH and simple steatosis, is important.

As a diagnostic method, liver biopsy is a standard method that has been conventionally performed. However, since liver biopsy is an invasive method of collecting tissues and cells by piercing the liver with a needle, alternative non-invasive methods are desired. As a non-invasive method, diagnosis by image inspection such as abdominal ultrasonic inspection, CT, and MRI is performed. However, the image diagnosis has limitations due to factors such as the body type of the subject, variations in results due to the skill and experience of the operator and the reader, and the scale, performance, and cost of each image diagnosis apparatus. It has been pointed out that the image diagnosis may not be suitable for screening a large group of subjects.

In order to solve these problems, a method for diagnosing NAFLD by collecting a body fluid of a patient and measuring metabolites therein has been proposed. For example, Patent Document 1 discloses a method for determining the amount of a specific lipid metabolite in a body fluid of a subject and correlating the amount with the presence of NAFLD. Patent Document 2 discloses a method for determining the amount of acetaminophen-glucuronide in a plasma sample of a subject to whom acetaminophen has been administered, and comparing the amount with a control. Patent Document 3 discloses a method for evaluating liver disease by measuring the concentration of soluble LR11 in a blood-derived sample. Patent Document 4 discloses a method of using the full length of high molecular weight kininogen and/or a partial peptide derived from high molecular weight kininogen as a biomarker. However, the methods disclosed in Patent Documents 1 to 4 have insufficient reliability because the number of samples of the subject is not as large as several tens or less.

Therefore, Patent Document 5 proposes a more reliable NAFLD diagnosis method based on data results from a larger number of samples.

PRIOR ART DOCUMENTS

PATENT DOCUMENTS

Patent Document 1: JP 2010-500566 W
Patent Document 2: JP 2012-504629 W
Patent Document 3: JP 2014-167446 A
Patent Document 4: WO2009/051259
Patent Document 5: WO2020/066162

SUMMARY OF THE INVENTION

PROBLEMS TO BE SOLVED BY THE INVENTION

However, in the method of Patent Document 5, since the area under the curve (AUC) of a receiver operating characteristic (ROC) curve is around 0.750 to 0.650, further improvement of the AUC is desired. That is, a method for identifying NAFLD with more excellent identification performance is desired.

It is an object of the present invention to provide a method for identifying NAFLD with excellent identification performance and a biomarker for use in the method.

MEANS FOR SOLVING THE PROBLEMS

An identification method for nonalcoholic fatty liver disease according to a first aspect of the present invention includes: an acquisition step of acquiring a sample derived from a body fluid of a subject; a detection step of detecting eight kinds of compounds consisting of alanine, isoleucine, glycine, serine, glutamic acid, asparagine, tyrosine, and uric acid in the sample; and a use step of using all detection amounts of the eight kinds of compounds obtained in the detection step.

EFFECTS OF THE INVENTION

The identification method and the biomarker according to the first aspect of the present invention have excellent identification performance of NAFLD.

BRIEF DESCRIPTION OF THE DRAWINGS

FIG. 1 is a graph evaluating the performance of a morbidity discriminant in Example 1 (13 kinds of specific compounds) using an ROC curve, and the right figure is a graph in which a sample cohort is a training set while the right figure is a graph in which a sample cohort is a test set. In FIG. 1, the vertical axis represents sensitivity, and the horizontal axis represents non-specificity (1-specificity).
FIG. 2 is a graph evaluating the performance of the morbidity discriminant in Examples using an ROC curve for a cohort different from FIG. 1.
FIG. 3 is a graph evaluating the performance of a morbidity discriminant in Example 2 (11 kinds of specific compounds) using an ROC curve, and the right figure is a graph in which a sample cohort is a training set while the right figure is a graph in which a sample cohort is a test set.
FIG. 4 is a graph evaluating the performance of a morbidity discriminant in Example 3 (eight kinds of specific compounds) using an ROC curve, and the right figure is a graph in which a sample cohort is a training set while the right figure is a graph in which a sample cohort is a test set.

MODE FOR CARRYING OUT THE INVENTION

1. First Embodiment

A first embodiment of the present invention is an identification method for NAFLD, in which all eight kinds of compounds consisting of alanine, isoleucine, glycine, serine, glutamic acid, asparagine, tyrosine, and uric acid are used as biomarkers for identifying NAFLD. Specifically, the identification method of the first embodiment includes an acquisition step, a detection step, and a use step. Hereinafter, each step will be described in detail.

(Acquisition Step)

In this step, a sample derived from a body fluid of a subject is obtained.
Examples of the body fluid include blood, urine, saliva, sweat, sputum, and feces. The blood includes whole blood, plasma, serum, and the like. The body fluid is preferably blood, more preferably serum. Blood sampling is minimally invasive, and blood is readily available as a sample to be subjected to screening in medical examinations and the like.
The sample is temporarily stored before the detection step. The storage method may be any of frozen storage, refrigerated storage, and room-temperature storage. When serum or plasma is used as the sample, it may be stored in the form of whole blood, or in the form of serum or plasma obtained by centrifuging whole blood.

(Detection Step)

In this step, at least all of the eight kinds of compounds consisting of alanine, isoleucine, glycine, serine, glutamic acid, asparagine, tyrosine, and uric acid are detected from the sample. Hereinafter, these eight kinds of compounds are also collectively referred to as specific compounds.
In this embodiment, the phrase "detecting a compound" refers to directly or indirectly detecting a specific compound contained in a sample for quantification, and specifically includes not only detecting the specific compound itself but also performing treatment such as ionization, dissociation, and derivatization on the specific compound to detect the treated specific compound (e.g., ionized matter, dissociated matter, or derivative).
The detection method may be any method capable of quantifying a specific compound contained in the sample, and examples thereof include gas chromatography (GC), liquid chromatography (LC), mass spectrometry (MS), gas chromatography/mass spectrometry (GC/MS), liquid chromatography/mass spectrometry (LC/MS), nuclear magnetic resonance spectroscopy, and immunological detection. From the viewpoint of enabling detection with high sensitivity for a blood sample, the detection method preferably involves the use of mass spectrometry (MS, LC/MS, or GC/MS), more preferably GC/MS.

When mass spectrometry is employed, examples of ionization means include electron ionization (EI), electrospray ionization (ESI), and atmospheric pressure chemical ionization (APCI). Examples of mass separation means include a quadrupole type, a magnetic sector type, a time-of-flight type, an ion trap type, and an ion cyclotron resonance type. Further examples thereof include a tandem mass separation type such as a triple quadrupole type (Q-Q), a tandem time-of-flight type (TOF-TOF), a quadrupole-time-of-flight type (Q-TOF), a quadrupole-ion trap type (Q-IT), a quadrupole-ion cyclotron resonance type (Q-ICR), and an ion trap-time-of-flight type (IT-TOF).

The sample may be subjected to pretreatment before the detection step is performed. Examples of the pretreatment include treatments for purification, derivatization, and the like. For example, when detection is performed by mass spectrometry using serum or the like, deproteinization treatment, derivatization, or the like is preferably performed.

The deproteinization treatment is performed, for example, by mixing each of an extraction solvent such as a chloroform solvent, water, and the like with a sample, centrifuging the mixture, and removing a precipitate of the mixture after centrifugation.

Examples of the derivatization include silylation, such as trimethylsilylation, dimethylsilylation, and halomethylsilylation; esterification such as methyl esterification; acylation; and alkylation. From the viewpoints of derivatizing a wide range of target compounds, reacting with many compounds containing active hydrogen, and producing a peak that facilitates mass spectrometry analysis, the derivatization is preferably silylation, more preferably trimethylsilylation. That is, it is preferable to use the detection amount of a derivative of the specific compound, particularly, the trimethylsilylated derivative of the specific compound.

When trimethylsilylation is employed, oximation may be performed, for example, by adding a methoxyamine solution to a freeze-dried sample, followed by the addition of a trimethylsilylating agent. Examples of the trimethylsilylating agent include N,O-bis(trimethylsilyl)trifluoroacetamide (BSTFA) and N-methyl-N-trimethylsilyltrifluoroacetamide (MSTFA).

Examples of the trimethylsilylated derivative of the specific compound include:

bis(trimethylsilyl)alanine (Alanine-2TMS),
bis(trimethylsilyl)isoleucine (Isoleucine-2TMS),
tris(trimethylsilyl)glycine (Glycine-3TMS),
tris(trimethylsilyl)serine (Serine-3TMS),
tris(trimethylsilyl)glutamic acid (Glutamic acid-3TMS),
tris(trimethylsilyl)asparagine (Asparagine-3TMS),
tris(trimethylsilyl)tyrosine (Tyrosine-3TMS), and
tetrakis tris(trimethylsilyl)uric acid (Uric acid-4TMS). When a specific compound is derivatized, a plurality of derivatives (e.g., Alanine-2TMS and Alanine-3TMS) may be obtained for one specific compound (e.g., Alanine). In such a case, only one of the plurality of derivatives may be arbitrarily selected, or a plurality of derivatives may be selected. When a plurality of derivatives is selected, a predetermined coefficient (described later) is set for each of the plurality of derivatives in a use step described later. In addition, when the coefficient is set, the detection amounts of a plurality of derivatizations may be added up. Then, the detection amounts of the plurality of derivatives and a plurality of predetermined coefficients corresponding thereto are substituted into the morbidity discriminant. Even when a plurality of derivatives is used from one specific compound, the number of specific compounds selected is treated as one.

During the pretreatment, it is preferable to mix stable isotopes of specific compounds and/or internal standard substances with the sample. The absolute amount of a specific compound contained in a body fluid, such as blood, can be measured by mixing stable isotopes. Further, the accuracy of quantification by mass spectrometry can be enhanced by mixing the internal standard substance. Examples of the stable isotopes include compounds in which one or more carbon atoms, hydrogen atoms, nitrogen atoms, or oxygen atoms in the molecule are substituted with a carbon stable isotope ($^{12}C$ or $^{13}C$), a hydrogen stable isotope ($^{1}H$, $^{2}H$, or D), a nitrogen stable isotope ($^{14}N$ or $^{15}N$), or an oxygen stable isotope ($^{16}O$, $^{17}O$, or $^{18}O$). The internal standard substance may be any substance that is not originally contained in the body fluid, and examples thereof include 2-isopropylmalic acid, ribitol, and tropic acid.

The pretreated sample is detected using the detection method. The conditions of the detection method may be appropriately set with reference to conditions recommended for the apparatus for use in the detection method (e.g., a GC/MS apparatus or an LC/MS apparatus).

As a result, the detection amount of each of the specific compounds contained in the sample is obtained. The detected amount is appropriately determined according to the detection method and detection apparatus, and may be output, for example, as a concentration; as a peak area or peak intensity in a mass spectrum or mass chromatogram; or as a light intensity. In such a case, the detection amount may be converted. For example, when the detection amount of the specific compound is output as the peak area of the mass spectrum, the peak area may be converted into the concentration. That is, the "detection amount" in the first embodiment includes not only the detection amount directly output from the detection apparatus but also the amount obtained by converting the detection amount into another parameter. Examples of the conversion include known methods such as an internal standard method and an absolute calibration curve method, and the conversion may be performed using any means such as an analysis function attached to a detection apparatus, another analysis apparatus, or a manual method. In the first embodiment, it is preferable to employ the concentration as the detection amount, and it is particularly preferable to employ the concentration ($\mu$g) of the specific compound present in 1 mL of serum.

(Use Step)

In this step, at least all detection amounts of the eight kinds of compounds obtained in the detection step are used. Specifically, all of the detection amounts of the eight kinds of compounds in the specific compounds are used to determine (decide) whether or not the subject suffers from NAFLD based on the use result. This use allows for identifying the presence or absence of NAFLD in the subjects.

For example, detection amounts of all eight kinds of specific compounds detected from a sample of a subject are used in a predetermined formula, specifically, substituted into the morbidity discriminant. Subsequently, the result obtained from this analysis is utilized to assess the likelihood of the subject developing NAFLD in the future.

The morbidity discriminant includes a sum (specifically, the second term on the right side of formula (1) to be described below) obtained by adding a value obtained by multiplying each of the detection amounts of the eight kinds of specific compounds by a predetermined coefficient for each specific compound. Specifically, each detection amount of the compound (eight kinds) is multiplied by a specific coefficient determined in advance for each compound. As a result, eight values are obtained for each compound, and therefore, the values are summed to derive a sum, and the sum is substituted into the morbidity discriminant.

Examples of such a morbidity discriminant include formulae (1) and (2) below.

[Equation 1]

$$[\text{Determination score}] = [\text{Constant K}] + \sum ([\text{Coefficient of each compound}] \times [\text{Detection amount of each compound}]) \cdots (1)$$

$$[\text{Probability of morbidity}] = 1 / (1 + e^{-[\text{Determination score}]}) \quad \cdots (2)$$

Then, the calculation result by the morbidity discriminant, that is, the probability of morbidity is used as a reference of whether or not the subject suffers from NAFLD. For example, the probability of morbidity indicates a probability of suffering from NAFLD in the range of 0 to 100%.

Note that the constant K and the predetermined coefficient (eight kinds of coefficients) of each compound are obtained, for example, by a method of acquiring data obtained by measuring a large number of samples of healthy individuals and samples of NAFLD patients in advance, and calculating and verifying the data using a statistical machine learning method (LASSO or the like). In the first embodiment, the inventors have already collected data from a large number of healthy individuals and NAFLD patients and calculated the results, which are listed in Tables 2 to 4 of Examples described later, and these constants K and the coefficient for each compound may be used.

In addition, as a method other than using the probability of morbidity as calculated according to formulae (1) and (2), for example, (i) a value using the detection amount of a specific compound detected from a sample of a subject (value of a subject) is compared with a value using the detection amount of a specific compound detected from a sample of a healthy individual or an NAFLD patient (value of a healthy individual or an NAFLD patient), and (ii) on the basis of whether the value of a subject is higher or lower than the value of a healthy individual or an NAFLD patient, it may be determined whether or not the subject suffers from NAFLD. Specifically, (i) a value obtained by substituting the detection amount of the subject into the second term on the right side of formula (1) is compared with a value obtained by substituting the detection amount of a healthy individual or an NAFLD patient into the second term on the right side of the formula (1). Then, (ii) when the value of the subject is equal to or higher (or lower) than the value of the NAFLD patient (threshold of NAFLD morbidity), the subject may be determined to be suffering from NAFLD, and when the value of the subject is equal to or lower (or higher) than the value of the healthy individual (normal threshold), the subject may be determined not to be suffering from NAFLD. A part or all of the present use step may be processed by a computer.

The identification method of the first embodiment enables determination of the presence or absence of NAFLD in the subjects with excellent identification performance, that is, accurate determination. As a result, the presence or absence of NAFLD in the subjects is accurately identified. Therefore, for example, when a medical practitioner presents a determination result by performing this identification method to a doctor, the doctor can use the determination result as a reference for confirming whether or not a medical examinee suffers from NAFLD, and can use the determination result for a subsequent treatment policy. Specifically, the treatment step may be performed after the identification method, particularly after the use step. The treatment step includes a known method in the treatment of NAFLD, and examples thereof include dietary therapy, exercise therapy, drug therapy, and surgical therapy. Diet therapy, exercise therapy, surgical therapy, and the like, which are aimed at weight reduction, are effective, and as the medication used in drug therapy, for example, antioxidants, diabetes therapeutic agents, and the like are effective. These therapies are applied in appropriate combination according to the situation of the subject.

2. Other Embodiments

In the first embodiment, the detection results of eight kinds of specific compounds are used, but the present invention only needs to include the detection amounts of eight kinds of specific compounds, and for example, the detection amounts of other compounds may also be added. That is, detection amounts of nine or more kinds of compounds may be used. Examples will be given below.

2-1. 13 Kinds of Specific Compounds

In the present embodiment, as a biomarker for identifying NAFLD, 2-hydroxybutyric acid, proline, decanoic acid, $\alpha$-ketoglutaric acid, and glucose are further used in addition to eight kinds of compounds consisting of alanine, isoleucine, glycine, serine, glutamic acid, asparagine, tyrosine, and uric acid. That is, 13 kinds of compounds consisting of alanine, isoleucine, glycine, serine, glutamic acid, asparagine, tyrosine, uric acid, 2-hydroxybutyric acid, proline, decanoic acid, $\alpha$-ketoglutaric acid, and glucose are used as biomarkers for identifying NAFLD. These 13 kinds of compounds are also collectively referred to as specific compounds.

This embodiment is the same as the above-described embodiment, except that these 13 kinds of compounds are used as biomarkers. That is, the identification method of the present implementation step includes the acquisition step, the detection step, and the use step as described above in the first embodiment. The present embodiment also exhibits the same advantageous effects as the first embodiment.

In the detection step, examples of trimethylsilylated derivatives of the 13 kinds of specific compounds include, in addition to the trimethylsilylated derivatives of the eight kinds of specific compounds, bis(trimethylsilyl)2-hydroxybutyric acid (2-hydroxybutyric acid-2TMS), bis(trimethylsilyl)proline (Proline-2TMS), trimethylsilyldecanoic acid (Decanoic acid-TMS), bis(trimethylsilyl)$\alpha$-ketoglutaric acid-methyloxime (a-Ketoglutaric acid-methyloxime-2TMS), and pentakis(trimethylsilyl)glucose-methyloxime (Glucose-methyloxime-5TMS). In the use step, the detection amounts of at least the 13 kinds of compounds are used. Specifically, values obtained by multiplying each detection amount (concentration or the like) of at least 13 kinds of specific compounds (or derivatives thereof) by a predetermined coefficient for each specific compound are added together, and the sum obtained thereby may be used for the morbidity discriminant.

2-2. 11 Kinds of Specific Compounds

In the present embodiment, as a biomarker for identifying NAFLD, lactic acid, succinic acid, and creatinine are further used in addition to eight kinds of compounds consisting of alanine, isoleucine, glycine, serine, glutamic acid, asparagine, tyrosine, and uric acid. That is, 11 kinds of compounds consisting of alanine, isoleucine, glycine, serine, glutamic acid, asparagine, tyrosine, uric acid, lactic acid, succinic acid, and creatinine are used as biomarkers for identifying NAFLD. These 11 kinds of compounds are also collectively referred to as specific compounds.

This embodiment is the same as the above-described embodiment, except that these 11 kinds of compounds are used as biomarkers. That is, the identification method of the present implementation step includes the acquisition step, the detection step, and the use step as described above in the first embodiment. The present embodiment also exhibits the same advantageous effects as the first embodiment.

In the detection step, examples of trimethylsilylated derivatives of the 11 kinds of specific compounds include, in addition to the trimethylsilylated derivatives of the eight kinds of specific compounds, bis(trimethylsilyl)lactic acid (Lactic acid-2TMS), bis(trimethylsilyl)succinic acid (Succinic acid-2TMS), and tris(trimethylsilyl)creatinine (Creatinine-3TMS). In the use step, the detection amounts of at least the 11 kinds of compounds are used. Specifically, values obtained by multiplying each detection amount of at least 11 kinds of specific compounds by a predetermined coefficient for each specific compound are added together, and the sum obtained thereby may be used for the morbidity discriminant.

3. Aspects

It is understood by those skilled in the art that the above-described illustrative embodiments are specific examples of the following aspects.

(Clause 1) An identification method for NAFLD according to an aspect may include: an acquisition step of acquiring a sample derived from a body fluid of a subject; a detection step of detecting eight kinds of compounds consisting of alanine, isoleucine, glycine, serine, glutamic acid, asparagine, tyrosine, and uric acid in the sample; and a use step of using all detection amounts of the eight kinds of compounds obtained in the detection step. This identification method enables determination of the presence or absence of NAFLD in the subjects with excellent identification performance. Therefore, the presence or absence of NAFLD is accurately identified.

(Clause 2) In the identification method according to clause 1, in addition to the eight kinds of compounds, lactic acid, succinic acid, and creatinine may be detected, and

in the use step, all detection amounts of 11 kinds of compounds obtained in the detection step may be used. This identification method enables more reliable determination of the presence or absence of NAFLD.

(Clause 3) In the identification method according to clause 1, in addition to the eight kinds of compounds, 2-hydroxybutyric acid, proline, decanoic acid, $\alpha$-ketoglutaric acid, and glucose may be detected, and in the use step, all detection amounts of 13 kinds of compounds obtained in the detection step may be used. This identification method enables more reliable determination of the presence or absence of NAFLD.

(Clause 4) In the identification method according to any one of clauses 1 to 3, in the use step, at least values obtained by multiplying each of the detection amounts of the eight kinds of compounds by a predetermined coefficient for each of the eight kinds of compounds may be summed. This identification method enables more reliable determination of the presence or absence of NAFLD.

(Clause 5) In the identification method according to any one of clauses 1 to 4, in the detection step, concentration of each of the eight kinds of compounds or a derivative thereof may be obtained. This identification method enables more reliable determination of the presence or absence of NAFLD.

(Clause 6) In the identification method according to clause 5, the derivative may be derivatized by silylation, acylation, esterification, or alkylation. Since the derivative can be detected with high sensitivity, this identification method enables more accurate determination of the presence or absence of NAFLD.

(Clause 7) In the identification method according to any one of clauses 1 to 6, wherein the detection step may be performed by gas chromatography, liquid chromatography, mass spectrometry, gas chromatography/mass spectrometry, liquid chromatography/mass spectrometry, nuclear magnetic resonance spectroscopy, or immunological detection. Since the detection method with good sensitivity is used, this identification method enables more accurate determination of the presence or absence of NAFLD.

(Clause 8) In the identification method according to any one of clauses 1 to 7, the body fluid may be blood. since the measurement sample is easily obtained and the invasiveness to the subject is low, this identification method can reduce the burden on the diagnosis of the subject.

(Clause 9) A biomarker for identifying NAFLD according to an aspect may contain eight kinds of compounds consisting of alanine, isoleucine, glycine, serine, glutamic acid, asparagine, tyrosine, and uric acid. This biomarker enables determination of the presence or absence of NAFLD in the subjects with excellent identification performance.

(Clause 10) The biomarker for identifying NAFLD according to clause 9 may contain 11 kinds of compounds consisting of alanine, isoleucine, glycine, serine, glutamic acid, asparagine, tyrosine, uric acid, lactic acid, succinic acid, and creatinine. This biomarker enables determination of the presence or absence of NAFLD in the subjects with excellent identification performance.

(Clause 11) The biomarker for identifying NAFLD according to clause 9 may contain 13 kinds of compounds consisting of alanine, 2-hydroxybutyric acid, isoleucine, proline, glycine, serine, decanoic acid, $\alpha$-ketoglutaric acid, glutamic acid, asparagine, glucose, tyrosine, and uric acid. This biomarker enables determination of the presence or absence of NAFLD in the subjects with excellent identification performance.

EXAMPLES

Next, the present invention will be described in detail with reference to Examples, but the scope of the present invention is not limited thereto.

<Example 1>

In the following examples, GC/MS is performed using a serum (NAFLD group) obtained from a patient diagnosed with NAFLD by abdominal ultrasound findings and a serum (normal group) obtained from a healthy individual, and the analysis result of searching for components in serum having different concentrations in the NAFLD group and the normal group will be described.

(Subject)

Among those randomly extracted from Japanese medical examinees who visited a hospital from October 2015 to September 2016, serum of 3,733 individuals who satisfied the eligibility criteria was used as a sample. The eligibility criteria required that subjects did not meet any of the conditions listed in (1) to (5) below.

(1) Person who has expressed disagreement with participation in study
(2) Persons whose alcohol intake is 30 g/day or more in terms of ethanol for males and 20 g/day or more in terms of ethanol for females
(3) person with present medical history during treatment of liver disease
(4) Person with present medical history during treatment for at least one of diabetes, dyslipidemia, and hyperuricemia
(5) Person with present medical history during treatment for malignant disease

All the individuals who satisfied the eligibility criteria had abdominal ultrasound findings in a complete medical checkup, and the results of analyzing clinical information and serum were cross-referenced under the consent of the Research Ethics Review Committee in the hospital. Of the 3,733 individuals in the NAFLD group and the normal group combined, there were 2,252 (60.3%) women, with a median age of 51 years (quartile range: 43 to 61 years old).

(Pretreatment)

After blood was collected from the subject, serum was separated by centrifugation, and frozen at -80°C within 6 hours from the blood collection. The serum after cryopreservation was left at 25°C for 5 minutes to thaw. Thawed serum was subjected to centrifugation, and the supernatant was collected in a tube containing a defined amount of 40 kinds of stable isotopes (Table 1 shows only stable isotopes corresponding to 13 kinds of metabolites among stable isotopes corresponding to 40 kinds of metabolites to be measured). To the collected serum, 250 $\mu$L of a mixed solvent of chloroform (methanol/water/chloroform = 2.5:1:1) and 6 $\mu$L of an internal standard solution (2-isopropylmalic acid: 0.1 mg/mL) were mixed and shaken to perform centrifugation. Subsequently, 140 $\mu$L of water was further mixed and shaken, and centrifugation was performed to obtain a sample containing an internal standard and a stable isotope. After being concentrated using a centrifugal evaporator under the condition of room temperature for 60 minutes, 180 $\mu$L of this sample was left at -80°C for 30 minutes to be frozen and was then dried overnight using a freeze dryer.

[Table 1]

| Stable Isotope |
|---|
| Sodium ($\pm$)-2-Hydroxybutyrate-2,3,3-d3 |
| Decanoic acid-1,2-13C2, 99 atom % 13C |
| $\alpha$-Ketoglutaric acid (13C5, 99%) |
| D-Glucose (13C6, 99%) |
| Uric acid (1,3-15N2, 98%) |
| L-Alanine (15N, 98%) |
| Glycine (15N, 98%) |
| L-Isoleucine (15N, 98%) |
| L-Proline (15N, 98%) |
| L-Serine (15N, 98%) |
| L-Glutamic acid (15N, 98%) |
| L-Asparagine·H2O (15N2, 98%) |
| L-Tyrosine (15N, 98%) |

(Derivatization)

The dried sample was sequentially mixed with 80 $\mu$L of a pyridine solution containing methoxyamine hydrochloride (20 mg/mL) and 40 $\mu$L of N-methyl-N-trimethylsilyltrifluoroacetamide, subjected to shaking, and then centrifuged, and 50 $\mu$L of the supernatant was transferred to an autosampler vial for GC/MS analysis.

(GC/MS Measurement)

Gas chromatography/mass spectrometry was performed on the sample under the following conditions.

<Conditions of Gas Chromatography>

System: GCMS-TQ8040 (manufactured by Shimadzu Corporation)
Column oven temperature: 80°C
Injection temperature: 280°C
Injection mode: Split injection method
Carrier gas: Helium
Carrier gas flow rate: 39 cm/sec
Separation column: DB-5 (manufactured by Agilent Technologies)
Stationary phase: (5%-phenyl)-methylpolysiloxane (nonpolar)
Column length: 30.0 m, column inner diameter: 0.25 mm, column film thickness: 1.00 $\mu$m
Column temperature program: 80°C (0 min)/80°C (2.5 min)/280°C (18.5 min)/280°C (23.0 min)

<Conditions of Mass Spectrometry >

System: GCMS-TQ8040 (manufactured by Shimadzu Corporation)
Ion source temperature: 200°C
Interface temperature: 250°C
Detector voltage: 0.2 kV
Measurement mode: Multiple Reaction Monitoring (MRM) mode
Scanning range: m/z 82-500

(Calculation of Detection Amount)

A mass spectrum at each retention time was output from the detection intensity obtained by scanning m/z at each retention time. Based on mass spectrum data in serum samples obtained in the past, metabolites (40 kinds) corresponding to each peak in the mass spectrum and stable isotopes corresponding to the metabolites were identified. The detection intensity corresponding to each metabolite was standardized using the detection intensity of the stable isotope corresponding to each metabolite, and the concentration (unit: $\mu$g/mL) in the sample was calculated as the detection amount.

(Data Analysis)

Based on the clinical diagnosis information and the detection amount obtained by GC/MS, a combination of metabolites capable of discriminating between the normal group and the onset group was examined using LASSO, which is one of machine learning techniques. Specifically, before the LASSO run, subjects were divided into two groups: a training set and a test set, and a morbidity discriminant was developed in the training set to perform the validation on the test set. In the LASSO run, logistic regression with 10-fold cross-validation implemented was applied.

As a result, as listed in Table 2 below, 13 kinds of compounds were selected as distinguishable metabolites, the regression coefficients thereof were calculated, and a morbidity discriminant was derived as described in the following formulae (1) and (2). In the morbidity discriminant, a formula obtained by adding a predetermined constant (the first term on the right side) and the sum of the respective compound detection amounts multiplied by the respective coefficients (the second term on the right side) is the determination score (the following formula (1)). Since this determination score corresponds to a logarithmic odds (expressed as ln{p/(1 - p)}, where p is defined as probability), the probability of morbidity (formula (2) below) is derived.

[Table 2]

| Compound | Coefficient |
|---|---|
| Constant K | -5.20539 |
| Alanine-2TMS | 1.61635 |
| 2-Hydroxybutyric acid-2TMS | 0.08041 |
| Isoleucine-2TMS | 0.42755 |
| Proline-2TMS | 0.03966 |
| Glycine-3TMS | -0.87309 |
| Serine-3TMS | -1.27909 |
| Decanoic acid-TMS | -1.26002 |
| $\alpha$-Ketoglutaric acid-methyloxime-2TMS | 1.53959 |
| Glutamic acid-3TMS | 3.29030 |
| Asparagine-3TMS | -0.42712 |
| Glucose-methyloxime-5TMS(3) | 0.86496 |
| Tyrosine-3TMS | 0.69018 |
| Uric acid-4TMS | 0.22084 |

[Equation 2]

$$[\text{Determination score}] = [\text{Constant K}] + \sum([\text{Coefficient of each compound}] \times [\text{Detection amount of each compound}]) \cdots (1)$$

$$[\text{Probability of morbidity}] = 1 / (1 + e^{-[\text{Determination score}]}) \quad \cdots (2)$$

TMS in Table 2 denotes the trimethylsilylated derivative. In formula 1, the second term on the right side is the sum of 13 kinds of compounds, the constant K and the coefficient of each compound are as listed in Table 2, and the detection amount of each compound was the concentration μg of the compound present in 1 mL of serum.

The resulting probability of morbidity was based on whether or not each subject currently suffers from NAFLD. Specifically, the probability of suffering from NAFLD is indicated by 0 to 100%.

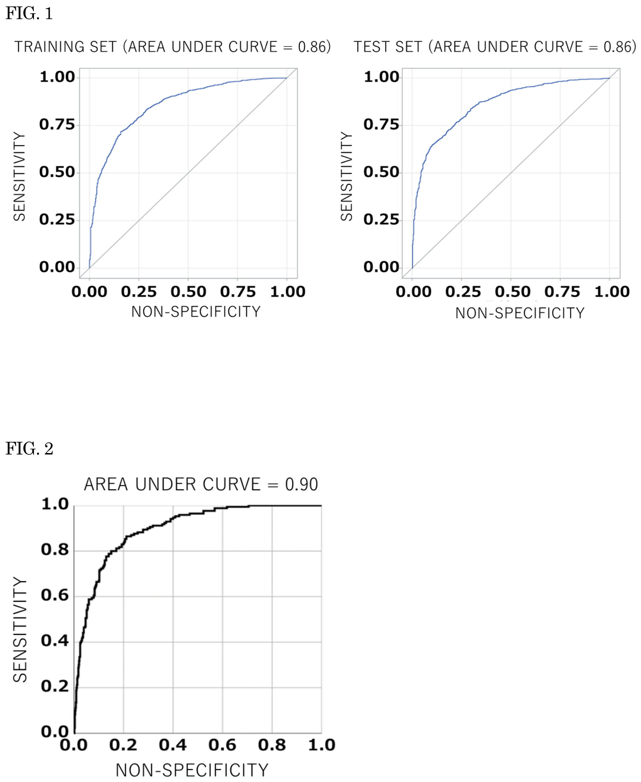

For the morbidity discriminant, the receiver operating characteristic (ROC) analysis was performed. The results are shown in FIG. 1. For quantitative evaluation, when the AUC at this time was calculated, the AUC was 0.86 in each of the training set and the test set, which was a very good value. Therefore, the identification method in the example demonstrated excellent identification performance. In addition, since it is based on data of 3,733 people, including a large number of healthy individuals and NAFLD patients, reliability is high.

In addition, as another cohort, among persons randomly extracted from Japanese medical examinees who visited a hospital at another time, serum of 899 persons who satisfied the above eligibility criteria was used as a sample. ROC analysis was also performed for this additional cohort and the results are shown in FIG. 2. The AUC at this time was 0.90.

<Example 2>

Values of 34 kinds of metabolites were subjected to the LASSO method in the same manner as in Example 1 (cohort of 3,733 people), except that six kinds of metabolites (Leucine-TMS, Decanoic acid-TMS, 1-Hexadecanol-TMS, Uridine-4TMS, 2-Aminoadipic acid-3TMS, and Histidine-3TMS) with unstable elements were excluded from the above 40 kinds of metabolites to enhance the stability of the analysis. As a result, as shown in Table 3, 11 kinds of compounds were selected as distinguishable metabolites, and regression coefficients thereof were calculated.

[Table 3]

| Compound | Coefficient |
|---|---|
| Constant K | -4.70150 |
| Lactic acid-2TMS | 0.11392 |
| Alanine-2TMS | 1.79441 |
| Isoleucine-2TMS | 0.18905 |
| Succinic acid-2TMS | -5.24464 |
| Glycine-3TMS | -1.09612 |
| Serine-3TMS | -1.25060 |
| Creatinine-3TMS | -0.14492 |
| Glutamic acid-3TMS | 2.51232 |
| Asparagine-3TMS | -1.10275 |
| Tyrosine-3TMS | 1.93978 |
| Uric acid-4TMS | 0.39709 |

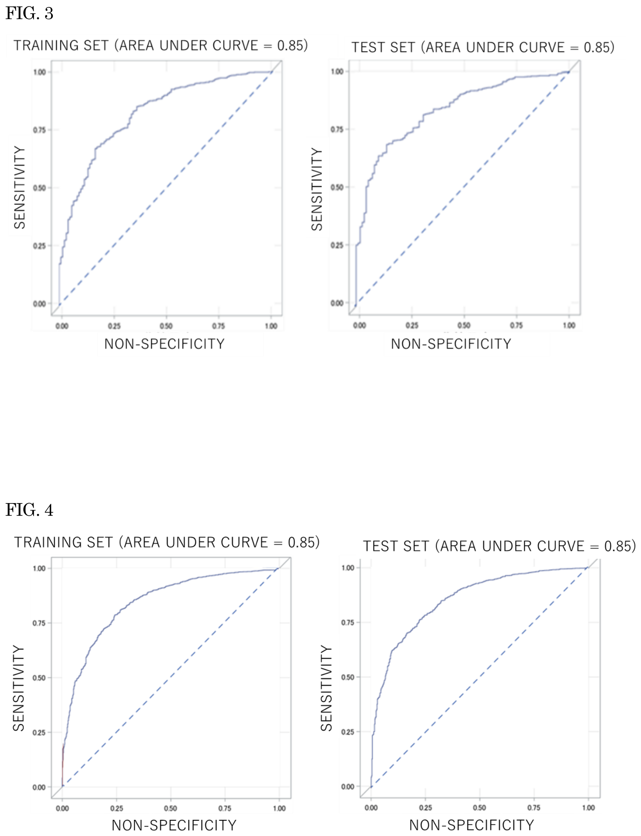

ROC analysis was performed using the 11 kinds of metabolites in the same manner as in Example 1, and the results are shown in FIG. 3. The AUCs at this time were both 0.85.

<Example 3>

The eight kinds of specific compounds common to 13 kinds of compounds of Example 1 and 11 kinds of compounds of Example 2 were selected as distinguishable metabolites (see Table 4 below).

[Table 4]

| Compound | Coefficient |
|---|---|
| Constant K | -4.70150 |
| Alanine-2TMS | 1.79441 |
| Isoleucine-2TMS | 0.18905 |
| Glycine-3TMS | -1.09612 |
| Serine-3TMS | -1.25060 |
| Glutamic acid-3TMS | 2.51232 |
| Asparagine-3TMS | -1.10275 |
| Tyrosine-3TMS | 1.93978 |
| Uric acid-4TMS | 0.39709 |

ROC analysis was performed using the 8 kinds of compounds in the same manner as in Example 1, and the results are shown in FIG. 4. The AUC was 0.85 for both, indicating strong predictive performance.

Claims

1.   An identification method for nonalcoholic fatty liver disease, the method comprising:

an acquisition step of acquiring a sample derived from a body fluid of a subject;
a detection step of detecting eight kinds of compounds consisting of alanine, isoleucine, glycine, serine, glutamic acid, asparagine, tyrosine, and uric acid in the sample; and
a use step of using all detection amounts of the eight kinds of compounds obtained in the detection step.

2. The identification method according to claim 1, comprising:

   detecting, in addition to the eight kinds of compounds, lactic acid, succinic acid, and creatinine in the detection step; and
   using, in the use step, all detection amounts of 11 kinds of compounds obtained in the detection step.

3. The identification method according to claim 1, comprising:

   detecting, in addition to the eight kinds of compounds, 2-hydroxybutyric acid, proline, decanoic acid, $\alpha$-ketoglutaric acid, and glucose in the detection step; and
   using, in the use step, all detection amounts of 13 kinds of compounds obtained in the detection step.

4. The identification method according to claim 1, comprising, in the use step, at least summing values obtained by multiplying each of the detection amounts of the eight kinds of compounds by a predetermined coefficient for each of the eight kinds of compounds.

5. The identification method according to claim 1, comprising, in the detection step, obtaining concentration of each of the eight kinds of compounds or a derivative thereof.

6. The identification method according to claim 5, wherein the derivative is derivatized by silylation, acylation, esterification, or alkylation.

7. The identification method according to claim 1, wherein the detection step is performed by gas chromatography, liquid chromatography, mass spectrometry, gas chromatography/mass spectrometry, liquid chromatography/mass spectrometry, nuclear magnetic resonance spectroscopy, or immunological detection.

8. The identification method according to claim 1, wherein the body fluid is blood.

9. A biomarker for identifying nonalcoholic fatty liver disease, the biomarker comprising eight kinds of compounds consisting of alanine, isoleucine, glycine, serine, glutamic acid, asparagine, tyrosine, and uric acid.

10. The biomarker according to claim 9, comprising 11 kinds of compounds consisting of alanine, isoleucine, glycine, serine, glutamic acid, asparagine, tyrosine, uric acid, lactic acid, succinic acid, and creatinine.

11. The biomarker according to claim 9, comprising 13 kinds of compounds consisting of alanine, 2-hydroxybutyric acid, isoleucine, proline, glycine, serine, decanoic acid, $\alpha$-ketoglutaric acid, glutamic acid, asparagine, glucose, tyrosine, and uric acid.

FIG. 1

TRAINING SET (AREA UNDER CURVE = 0.86)    TEST SET (AREA UNDER CURVE = 0.86)

FIG. 2

AREA UNDER CURVE = 0.90

FIG. 3

TRAINING SET (AREA UNDER CURVE = 0.85)    TEST SET (AREA UNDER CURVE = 0.85)

FIG. 4

TRAINING SET (AREA UNDER CURVE = 0.85)    TEST SET (AREA UNDER CURVE = 0.85)

## INTERNATIONAL SEARCH REPORT

| International application No. |
|---|
| **PCT/JP2024/017844** |

| A. | CLASSIFICATION OF SUBJECT MATTER |
|---|---|

*G01N 33/68*(2006.01)i
FI:  G01N33/68

According to International Patent Classification (IPC) or to both national classification and IPC

| B. | FIELDS SEARCHED |
|---|---|

Minimum documentation searched (classification system followed by classification symbols)

G01N33/68

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2020/066162 A1 (SHIMADZU CORPORATION) 02 April 2020 (2020-04-02) paragraphs [0020]-[0027], [0034]-[0056] | 1-2, 4-10 |
| Y | | 3, 11 |
| Y | JP 2018-502286 A (METABOLON, INC.) 25 January 2018 (2018-01-25) paragraphs [0119]-[0120] | 1-11 |
| Y | 篁俊成，1．病態解明・診断・治療　1．非アルコール性脂肪性肝疾患，日本内科学会雑誌, 10 April 2013, vol. 102, no. 4, pp. 836-844, (TAKANURA, Toshinari. I. Pathophysiology, Diagnosis and Treatment 1. Non-alcoholic Fatty Liver Disease. Nihon Naika Gakkai Zasshi.) in particular, see "Introduction" | 1-11 |
| Y | WO 2013/002381 A1 (AJINOMOTO CO., INC.) 03 January 2013 (2013-01-03) paragraphs [0017], [0021], [0027], [0080], [0084], [0101], [0127], [0273], [0276], [0282] | 1-11 |
| A | JP 2013-040923 A (AJINOMOTO CO., INC.) 28 February 2013 (2013-02-28) paragraphs [0015], [0050], claims | 1-11 |

[✓] Further documents are listed in the continuation of Box C.  [✓] See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **11 June 2024** | **25 June 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

# EP 4 715 384 A1

| INTERNATIONAL SEARCH REPORT | International application No. |
|---|---|
| | **PCT/JP2024/017844** |

**C.    DOCUMENTS CONSIDERED TO BE RELEVANT**

| Category* | Citation of document, with indication, where appropriate, of the relevant passages | Relevant to claim No. |
|---|---|---|
| A | LAI, Yisyuan et al. Mass-spectrometry-based serum metabolomics of a C57BL/6J mouse model of high-fat-diet-induced non-alcoholic fatty liver disease development. Journal of agricultural and food chemistry. 09 September 2015<br>abstract, p. 7879, potential biomarkers and metabolic pathways, p. 7881, table 3, fig. 6 | 1-11 |
| A | WO 2006/129513 A1 (AJINOMOTO CO., INC.) 07 December 2006 (2006-12-07)<br>    claims | 1-11 |
| A | WO 2013/011919 A1 (AJINOMOTO CO., INC.) 24 January 2013 (2013-01-24)<br>    claims, paragraph [0050] | 1-11 |
| A | JP 2016-65873 A (METANOMICS HEALTH GMBH) 28 April 2016 (2016-04-28)<br>    paragraphs [0112]-[0113], table 1A | 1-11 |

**INTERNATIONAL SEARCH REPORT**
Information on patent family members

International application No.

**PCT/JP2024/017844**

| Patent document cited in search report | | | Publication date (day/month/year) | Patent family member(s) | | | Publication date (day/month/year) |
|---|---|---|---|---|---|---|---|
| WO | 2020/066162 | A1 | 02 April 2020 | (Family: none) | | | |
| JP | 2018-502286 | A | 25 January 2018 | (Family: none) | | | |
| WO | 2013/002381 | A1 | 03 January 2013 | US in particular, abstract, claims | 2014/0113378 | A1 | |
| JP | 2013-040923 | A | 28 February 2013 | US abstract, claims | 2014/0127819 | A1 | |
| WO | 2006/129513 | A1 | 07 December 2006 | US EP | 2008/0154515 1887362 | A1 A1 | |
| WO | 2013/011919 | A1 | 24 January 2013 | US | 2014/0127819 | A1 | |
| JP | 2016-65873 | A | 28 April 2016 | US EP | 2013/0126722 2863227 | A1 A1 | |

Form PCT/ISA/210 (patent family annex) (July 2022)

**EP 4 715 384 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010500566 W
- JP 2012504629 W
- JP 2014167446 A
- WO 2009051259 A
- WO 2020066162 A